# EUROPEAN PATENT SPECIFICATION

(11) **EP 1 163 912 B1**
(45) Date of publication and mention of the grant of the patent: **31.10.2007**
(21) Application number: 00908055.7
(22) Date of filing: 14.03.2000
(51) Int. Cl.: A61K 48/00, A61K 38/00

(54) **GENE THERAPEUTICS**
GENTHERAPEUTIKA
THERAPEUTIQUE GENIQUE

(30) Priority: 23.03.1999 JP 7859199
(43) Date of publication of application: 19.12.2001
(73) Proprietor: TAKARA BIO INC., Otsu-shi, Shiga (JP)
(72) Inventor: KATO, Ikunoshin, Uji-shi, Kyoto 611-0028 (JP); ASADA, Kiyozo, Koka-gun, Shiga 520-3333 (JP); UENO, Mitsuhiro, Kusatsu-shi, Shiga 525-0025 (JP); HASHINO, Kimikazu, Takatsuki-shi, Osaka 569-0082 (JP); YOSHIOKA, Hirofumi, 311, Hamoparesu-Kusatsu, Kusatsu-shi, Shiga 525-025 (JP); TANAKA, Keiji, Otsu-shi, Shiga 520-0821 (JP)
(74) Representative: Schnappauf, Georg
(86) International application number: PCT/JP2000/001533
(87) International publication number: WO 2000/056368

(56) References cited:
- EP-A1- 0 870 839
- WO-A1-00/01836
- WO-A1-95/26200
- WO-A1-95/31566
- WO-A1-97/31656
- JP-A- 10 505 234
- JP-A- 10 507 074
- US-A- 5 830 880
- NAMBA H ET AL: "Bystander effect-mediated therapy of experimental brain tumor by genetically engineered tumor cells." HUMAN GENE THERAPY. UNITED STATES 1 JAN 1998, vol. 9, no. 1, 1 January 1998 (1998-01-01), pages 5-11, XP009011552 ISSN: 1043-0342
- HANENBERG H ET AL: "COLOCALIZATION OF RETROVIRUS AND TARGET CELLS ON SPECIFIC FIBRONECTIN FRAGMENTS INCREASES GENETIC TRANSDUCTION OF MAMMALIAN CELLS" NATURE MEDICINE, NATURE PUBLISHING, CO, US, vol. 2, no. 5, August 1996 (1996-08), pages 876-882, XP002928787 ISSN: 1078-8956
- HARRINGTON KEVIN ET AL: "Cells as vehicles for cancer gene therapy: the missing link between targeted vectors and systemic delivery?" HUMAN GENE THERAPY. UNITED STATES 20 JUL 2002, vol. 13, no. 11, 20 July 2002 (2002-07-20), pages 1263-1280, XP002242755 ISSN: 1043-0342
- MAKOTO MIGITA et al., "Retrovirus vector ni yoru zouketsu kansaibou e no idenshi dounyuu no shiteki jouken no kentou - kotsuzui stroma saibou to fibronectin fragment (CH-296) no hikaku-", Kokuritsu seishin. shinkei center uneibu kikakushitsu ed., "Heisei 9 nendo kouseishou seishin. shinkei shikkan kenkyuu houkokushuu (2 nendo han. shonendo han)", 1998, page 401, XP002946283
- HANENBERG H. ET AL.: 'Colocalization of retrovirus and target cells on specific fibronectin fragments increases genetic transduction of mammalian cells' NATURE MEDICINE vol. 2, no. 6, 1996, pages 876 - 882, XP002928787
- IKUNOSHIN KATO: 'Fibronectin no riyou ni yoru zouketsu kansaibou e no kou kouritsu idenshi dounyuuhou' IDENSHI IGAKU vol. 3, no. 2, May 1999, pages 114 - 119, XP002946280

## Description

### Technical Field

The present invention relates to a composition and a method for missile gene therapy which are useful for treatment of diseases that require gene therapy for their treatment and for selective transfer of a gene into target cells in vivo.

### Background Art

About 3000 cases of gene therapies have been conducted in the world to date. The greatest technical problem concerning the gene therapy was that the efficiency of transferring a therapeutic gene into target cells, particularly into hematopoietic stem cells, is very low. Recently, the gene transfer efficiency has been remarkably improved by the use of a recombinant protein of a fibronectin fragment, CH-296 (Takara Shuzo; RetroNectin), and thus the gene therapy is gaining practicality (Nature Medicine, 2:876-882 (1996)). The recombinant RetroNectin molecule can bind both a retrovirus having a therapeutic gene being incorporated and a target cell to allow them to become adjacent each other, thereby greatly increasing the gene transfer efficiency. It has been said that it is the most difficult to transfer a gene into human hematopoietic stem cells. However, transfer of a therapeutic gene with an efficiency of about 90% has been achieved using RetroNectin even for the human hematopoietic stem cells. RetroNectin is a single polypeptide in which a peptide that specifically binds to a hematopoietic stem cell is connected to a peptide that specifically binds to a retrovirus vector having a therapeutic gene being incorporated. The present inventors have demonstrated that the two portions in RetroNectin exhibit the same activity as that of the original RetroNectin molecule even if they are separated each other and mixed as a cocktail, and designated this method as a cocktail gene transfer method (see WO 97/18318).

The method for transferring a gene into hematopoietic stem cells using RetroNectin is an epoch-making method in that it increases the efficiency of transferring a gene into hematopoietic stem cells. This method comprises conducting gene transfer into hematopoietic stem cells in vitro and returning the hematopoietic stem cells having the transferred gene into a living body. Thus, it may be considered that the method comprising such steps is too complicated to apply it to gene therapy in some cases.

Currently, there is a need to provide a target cell-specific gene transfer method which can deal with the diversity of target cells to be used for gene therapy.

Attempts have been made to confer directivity to target cells on non-virus vectors (e.g., a vector that uses polylysine or the like as a carrier for retaining a nucleic acid) by adding a ligand having an affinity specific for the cells. However, a gene transferred by this method cannot be stably maintained in cells. Virus vectors each expressing a fusion protein of a viral envelope with a ligand having an affinity for a target cell are known. However, the intended targeting has not been accomplished in many cases because one or both of the infective function inherent in the envelop and the binding function inherent in the ligand is damaged due to the expression as a fusion. Furthermore, it was necessary to carry out complicated construction of packaging cells for every type of target cell in order to express the fused envelope. In addition, a lot of time for preparing experiments has been required to establish a packaging cell line that can provide a high-titer virus vector suspension, to confirm that a replication competent retrovirus (RCR) does not appear, and the like. Namba et al. (Human Gene Therapy 9:5-11, 1998) describe the bystander effect-mediated therapy of experimental brain tumor by genetically engineered tumor cells.

As described above, it has been desired to solve various problems still associated with the current techniques in order to efficiently transfer a gene of interest specifically into target cells.

### Objects of Invention

The main object of the present invention is to provide a therapeutic composition useful for gene therapy comprising transferring a gene in vivo, and to provide a convenient gene therapy method comprising transferring a gene specifically into target cells in vivo using said therapeutic composition.

### Summary of Invention

The first aspect of the invention relates to a composition for gene therapy used for treating a disease susceptible to gene therapy, which contains as active ingredients an effective amount of a retrovirus that contains a gene useful for gene therapy as well as an effective amount of a cell as a vehicle that has an affinity for the retrovirus and an affinity specific for a target cell for which transfer of the gene is required, wherein the affinity for the retrovirus is derived from a functional substance selected from the group consisting of anti-virus antibodies, heparin-II-binding domain of fibronectin, fibroblast growth factor, collagen and polylysi. and is conferred by binding said functional substance to the cell as a vehicle or by expressing said functional substance on the surface of the cell as a vehicle.

In one embodiment of the first aspect of the invention the affinity for the target cell is conferred by forcing the cell as a vehicle to express a functional substance having the affinity for the target ceil on said cell as a vehicle, wherein said functional substance is a ligand for a receptor expressed on the target cell or an antibody against a surface antigen on the target cell.

In another embodiment of the first aspect of the invention the cell as a vehicle is selected from the group consisting of vascular endothelial cells, inflammatory cells, hematopoietic stem cells, brain endothelial cells and bone marrow cells.

The second aspect of the invention relates to the use of an effective amount of a cell as a vehicle that has an affinity for a retrovirus that contains a gene useful for gene therapy and an affinity specific for a target, cell for which transfer of the gene is required for the preparation of a pharmaceutical composition for gene therapy for treating a disease susceptible to gene therapy, wherein the affinity for the retrovirus is derived from a functional substance selected from the group consisting of anti-virus antibodies, heparin-II-binding domain of fibronectin, fibroblast growth factor, collagen and polylysine, and is conferred by binding said functional substance to the cell as a vehicle or by expressing said functional substance on the surface of the cell as a vehicle.

In one embodiment of the second aspect of the invention the affinity for the target cell is conferred by forcing the cell as a vehicle to express a functional substance having the affinity for the target cell on said cell as a vehicle, wherein said functional substance is a ligand for a receptor expressed on the target cell or an antibody against a surface antigen en the target cell.

In another embodiment of the second aspect of the invention the composition contains an effective amount of the retrovirus that contains a gene useful for gene therapy.

In even another embodiment of the second aspect of the invention the cell as a vehicle is selected from the group consisting of vascular endothelial cells, inflammatory cells, hematopoietic stem cells, brain endothelial cells and bone marrow cells.

In another embodiment of the first or second aspect of the invention the target cell is a cell selected from the group consisting of hematopoietic stem cells, blood cells, leukocytes, lymphocytes, T cells, tumor-infiltrating lymphocytes, B cells and cancer cells.

In even another embodiment of the first or second aspect of the invention the gene useful for gene therapy encodes a therapeutic protein which is expressed upon expression of the gene in the target cell in an amount sufficient for the treatment.

In another embodiment of the first or second aspect of the invention the therapeutic protein is an enzyme or a cytokine.

Thus, the present invention has been completed.

### Brief Description of Drawings

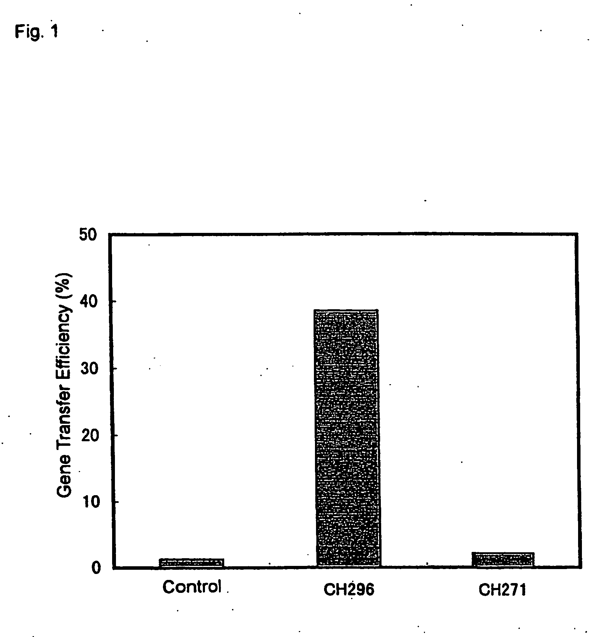

Figure 1 illustrates the efficiency of gene transfer using HL-60 cell as a vehicle.

### Detailed Description of the Invention

The retro virus vector used in the missile gene therapy using the therapeutic composition of the present invention is not specifically limited. Know retro virus vectors usually used for gene transfer are used. A recombinant retrovirus vector is preferably used in the present invention. In particular, a replication-defective recombinant retrovirus vector is preferable. The ability of replication of such a vector is eliminated such that it cannot autonomously replicate in infected cells and, therefore, the vector is non-pathogenic. The vector can invade into a host cell such as a vertebrate cell (particularly, a mammalian cell) and stably integrate a foreign gene useful for gene therapy inserted within the vector into the chromosomal DNA.

In the present invention, the gene to be transferred into target cells in vivo can be used being inserted into a recombinant retrovirus vector such that it is expressed under the control of an appropriate promoter, for example, a promoter present in the virus vector or a foreign promoter. In addition, another regulatory element (e.g., an enhancer sequence or a terminator sequence) that cooperates with a promoter and/or a transcription initiation site may be present in the vector in order to accomplish efficient transcription of the gene. Furthermore, a promoter, a transcription initiation site and another regulatory element cooperating with them that control expression in a site-specific manner (in an organ, around tumor, etc.) may be incorporated into the vector to further increase the specificity of gene expression at the target site. The gene to be transferred may be a naturally occurring gene or an artificially prepared gene. Alternatively, the gene may be one in which DNA molecules of different origins are joined together by ligation or other means known in the art.

One can select any gene of which the transfer into target cells in vivo is desired as the gene to be inserted into the virus vector. For example, a gene encoding an enzyme or a protein associated with the disease to be treated, an intracellular antibody (see, for example, WO 94/02610), a growth factor, an antisense nucleic acid, a ribozyme, a false primer (see, for example, WO 90/13641) or the like can be used as the gene.

Examples of functional substances having affinities for viruses include, but are not limited to, anti-virus antibodies, heparin-II-binding domain of fibronectin, fibroblast growth factor, type V collagen and polylysine. Also, substances functionally equivalent to these functional substances such as a functional substance having heparin-binding domain are disclosed. They may be derived from such functional substances. In this context, "derived from a functional substance" means that a functional site of a functional substance having an affinity for a virus is included in the molecule of the functional substance to be used. An affinity is a conception that includes an ability to bind to a virus and an ability to adhere to a cell. The affinity for a virus of the functional substance used in the present invention makes it possible to target a virus to a specific cell, or an organ or a tissue containing the cell, and to conduct gene therapy comprising transferring a gene in vivo into a specific cell.

An antibody that has an affinity specific for a retrovirus vector is particularly useful for specifically and efficiently transferring a gene into specific cells. The antibody that can be used in the present invention is not limited to a specific one. An antibody that recognizes an antigen on the surface of a retrovirus vector for gene transfer can be appropriately selected for use. Such an antibody can be produced according to known methods. Alternatively, many currently commercially available antibodies can also be used. The antibody may be either a monoclonal antibody or polyclonal a antibody as long as it has desired properties such as an ability to bind to a virus to be used. Additionally, an antibody or a derivative of an antibody modified using known techniques such as a humanized antibody, a Fab fragment or a single-chain antibody can also be used.

Also disclosed are examples of functional substances having affinities for target cells which include, but are not limited to, proteins each having an affinity for the cell, hormones, cytokines, antibodies against cell surface antigens, polysaccharides, glycoproteins, glycolipids, sugar chains derived from glycoproteins or glycolipids, metabolites of the target cells and cells. They may be cell-binding sites derived from such functional substances. In this context, "derived from a functional substance" means that a functional site of a functional substance having an affinity for a target cell is included in the molecule of the functional substance to be used. An affinity for a target cell is a conception that includes an ability to bind to a target cell and an ability to adhere to a cell. The affinity for a target cell of a functional substance makes it possible to target a virus to a specific cell, or an organ or a tissue containing the cell, and to conduct gene therapy comprising transferring a gene in vivo into a specific cell.

An antibody that specifically binds to a target cell is particularly useful for efficiently transferring a gene into specific target cells. The anti-target cell antibody that can be used in the present invention is not limited to a specific one. An antibody against an antigen expressed on target cells into which a gene is to be transferred can be appropriately selected for use. Such an antibody can be produced according to known methods. Alternatively, many currently commercially available antibodies can also be used. The antibody may be either a monoclonal antibody or a polyclonal antibody as long as it has desired properties such as specificity for the target cell. Additionally, an antibody or a derivative of an antibody modified using known techniques such as a humanized antibody, a Fab fragment or a single-chain antibody can also be used.

Expression of respective leukocyte antigens (known as CD antigens) on various cells has been studied in detail. Thus, a gene can be transferred into target cells with high specificity by selecting an antibody that recognizes a CD antigen expressed on the target cells of interest and using it in the gene transfer method of the present invention. For example, gene transfer can be directed to helper T cells by using an anti-CD4 antibody, or to hematopoietic stem cells by using an anti-CD34 antibody.

Furthermore, a protein having an activity of adhering to a cell such as fibronectin, laminin, collagen or vitronectin is disclosed as a functional substance having an affinity for a target cell. The functional substance may be a fragment thereof as long as it has an activity of binding to a target cell.

A glycoprotein, laminin, is disclosed for efficiently transferring a gene into various target cells such as blood cells. The sugar chain of laminin plays an important role in gene transfer using laminin. Therefore, a sugar chain released from laminin according to a known method is disclosed as a functional substance. Furthermore, a glycoprotein having a high mannose type N-linked sugar chain like laminin, or a sugar chain released therefrom or chemically synthesized is disclosed. Additionally, a substance, such as a protein, having the above-mentioned sugar chain being attached thereto is disclosed. For example, a functional substance having an affinity for a retrovirus and having the sugar chain being attached thereto is disclosed.

The functional substance as described above can be obtained from naturally occurring materials, prepared artificially (for example, using recombinant DNA techniques or chemical synthesis techniques), or prepared by combining a naturally occurring substance and an artificially prepared substance.

The heparin II-binding domain of fibronectin used in the method of the present invention can be prepared in a substantially pure form from naturally occurring materials according to methods as described, for example, in J. Biol. Chem., 256:7277 (1981); J. Cell. Biol., 102:449 (1986); or J. Cell. Biol., 105:489 (1987). The heparin II-binding domain of fibronectin can be prepared utilizing recombinant DNA techniques as described in United States Patent No. 5,198,423. Specifically, a fibronectin fragment containing heparin-II domain, which is a retrovirus-binding site, such as recombinant polypeptides including CH-296 (RetroNectin), H-271, H-296 and CH-271 as well as the method for obtaining them are described in detail in the publication of the patent. These fragments can be obtained by culturing Escherichia coli strains deposited under accession numbers FERM P-10721 (H-296) (the date of the original deposit: May 12, 1989), FERM BP-2799 (CH-271) (the date of the original deposit: May 12, 1989), FERM BP-2800 (CH-296) (the date of the original deposit: May 12, 1989) and FERM BP-2264 (H-271) (the date of the original deposit: January 30, 1989) at the National Institute of Bioscience and Human-Technology, Agency of Industrial Science and Technology, Ministry of International Trade and Industry, 1-3, Higashi 1-chome, Tsukuba-shi, Ibaraki-ken, Japan as described in the publication. In addition, fragments that can be typically derived from these fragments can be prepared by modifying the plasmids harbored in these Escherichia coli strains using known recombinant DNA techniques.

Among the above-mentioned fibronectin fragments, CH-296 and CH-271 have ligands for VLA-5, and CH-296 and H-296 have ligands for VLA-4. Thus, they are useful for targeting into cells expressing VLA-5 or VLA-4. For example, a functional substance having a ligand for VLA-4 is useful for transferring a gene into hematopoietic stem cells.

A cell is disclosed having an affinity to a target cell. Certain cells have affinities specific for organs, tissues or cells. Thus, the cell is useful as a vehicle for infecting a target cell with a virus vector in vivo. Targeting of a gene into a target cell using a cell as a vehicle is exemplified by the following.

### 1. Gene transfer using vascular endothelial cell as vehicle

A vascular endothelial cell has a nature of being accumulated specifically at a site at which a blood vessel is newly formed. Targeting of a gene using a vascular endothelial cell as a vehicle can be conducted utilizing this nature.

Cancer cells induce vascularization around them for their growth, take up nutrients and excrete wastes through the formed blood vessels. Cancer can be treated by transporting a virus vector to a site of vascularization around the cancer cells utilizing the nature of the vascular endothelial cell as described above. For example, a suicide gene such as HSV-TK may be transferred as a therapeutic gene to directly attack a cancerous tissue. Alternatively, a gene that inhibits vascularization may be transferred to inhibit the uptake of nutrients by cancer cells and regress the cancer. Side effect observed for such a therapy for cancer is less than that observed for conventional chemotherapy or radiotherapy. The physical burden to a patient due to surgery is dramatically reduced by using simple treatment comprising inoculation with the composition for gene therapy of the present invention.

It is preferable to promote the development of collateral circulation pathway in order to overcome the ischemic state observed after blood vessel bypass surgery for cerebral infarction or myocardial infarction. In this case, the ischemic state is ameliorated by transferring a gene involved in promotion of vascularization to a site of vascularization around the site of surgery using a vascular endothelial cell as a vehicle.

### 2. Gene transfer into inflamed tissue using inflammatory cell as vehicle

In case of allergic inflammation such as bronchial asthma, inflammatory cells from blood vessel lumen adhere to blood vessel wall, migrate through vascular endothelial cells, and then move into stroma to cause inflammation in respiratory tract mucous membrane. A therapy can be conducted utilizing this nature of being accumulated at an inflammation site of an inflammatory cell. Inflammatory cells that can be used as vehicles include eosinophils, mast cells and lymphocytes. For example, upon adhesion of inflammatory cells to blood vessel wall which is the first step of accumulation, if a gene involved in inhibition of adhesion is transferred into vascular endothelial cells, the adhesion of inflammatory cells is inhibited and the accumulation does not take place thereafter.

### 3. Gene transfer into bone marrow microenvironment using hematopoietic stem cell as vehicle

A hematopoietic stem cell has a nature of homing into bone marrow microenvironment. Targeting of a gene can be conducted utilizing this nature. When hematopoietic stem cells home into bone marrow microenvironment along with a virus vector, a useful gene can be transferred to bone marrow microenvironment including adjacent other hematopoietic stem cells and cells composing the bone marrow microenvironment such as stromal cells.

### 4. Gene transfer into brain tumor using brain endothelial cell as vehicle

Targeting to a site of brain tumor can be conducted by binding a virus vector to endothelial cells derived from brain. In particular, a retrovirus vector has a nature of infecting dividing cells with high efficiency. Thus, it can transport a healing gene specifically into brain tumor without infecting non-dividing normal cells surrounding tumor.

### 5. Gene transfer using cell capable of regenerating tissue as vehicle

Recently, regeneration of blood vessels using bone marrow cells was reported, and treatment of myocardial infarction utilizing this observation has been conducted. Blood flow in cardiac muscle in infarcted state is improved by injecting bone marrow cells into the cardiac muscle. If bone marrow cells are allowed to transport a virus vector having a useful gene (e.g., a vascularization-promoting gene) utilizing this nature, the ability of the bone marrow cells to regenerate blood vessel is dramatically increased due to synergistic effect with the transferred gene. Furthermore, bone marrow cells are capable of differentiating and regenerating into bone, cartilage, tendon, fat cells, skeletal muscle and stromal cells as mesenchymal stem cells are. Thus, bone marrow cells can be utilized for promotion of regeneration of tissues or cells, site-specific treatment and the like by using the bone marrow cells for transporting a therapeutic gene suitable for the purpose.

Functional substances are disclosed that include one composed of a functional substance having an affinity for a virus and a functional substance having an affinity for a target cell. For example, the functional substances are exemplified by a functional substance consisting of an anti-target cell antibody having an affinity specific for a target cell and an anti-virus antibody having an affinity specific for a retrovirus, a functional substance consisting of a sugar chain having an affinity specific for a target cell and an anti-virus antibody having an affinity specific for a retrovirus, and a functional substance consisting of a cell having an affinity specific for a target cell and a polypeptide having an affinity specific for a retrovirus. A gene transferred into specific cells in a living body in which various types of cells coexist using such a functional substance is disclosed. In particular, sugar chains are said to be the faces of cells because they determine diverse properties of cells and cells recognize and interact each other through various sugar chains. Thus, targeting of gene transfer utilizing this specificity of sugar chain enabling the most precise missile gene therapy in vivo when it is used along with an antibody which has a specific binding ability is disclosed.

The composition for gene therapy of the present invention using a cell as a vehicle is exemplified by one in which a functional substance having a affinity for a retrovirus vector is bound to a cell having an affinity specific for a target cell. Methods used for binding functional substances to cells include a method in which a functional substance is chemically bound to a cell and a method in which a functional substance that has both of an affinity for a virus vector and an affinity specific for a cell to be used as a vehicle is used.

Furthermore, a cell that has an affinity specific for a retrovirus vector and an affinity specific for a target cell can be utilized as a vehicle. Native cells that inherently have both of the above-mentioned properties are disclosed. Alternatively, a cell having one or both of the two affinities being artificially conferred is disclosed. An affinity specific for a target cell can be conferred by forcing the cell to express a functional substance having an affinity specific for the target cells (e.g., a ligand for a receptor expressed on the target cell or an antibody against a surface antigen on the target cell) on the surface of the cell. In addition, an affinity for a retrovirus vector can be similarly conferred by expressing a functional substance having an affinity specific for the retrovirus vector on the surface of the vehicle cell.

Vehicle cells prepared from the patient to be administered with the composition for gene therapy of the present invention are not eliminated by immunity or the like. Thus, they are particularly preferable for therapeutic purpose. If it is impossible to prepare vehicle cells from a patient, cells derived from another individual or another animal species may be used. In this case, if the cells are treated with radiation or a drug beforehand, the cells do not grow and disappear when cells begin to divide after a certain period of time from the administration into the living body. Such cells are sufficiently functional for the purpose of transporting a virus vector.

Additionally, gene transfer efficiency can be further increased by utilizing the interaction (e.g., signal transduction) between the vehicle cell and the target cell to produce a state in which the target cell becomes susceptible to infection with the retrovirus vector, for example, to progress cell cycle.

A composition for gene therapy used for treating a disease susceptible to gene therapy can be manufactured using as an active ingredient an effective amount of a cell as vehicle that has an affinity for a retrovirus that contains a gene useful for gene therapy and an affinity specific for a target cell for which transfer of the gene is required. Furthermore, a composition for gene therapy used for treating a disease susceptible to gene therapy can be manufactured using as active ingredients an effective amount of a functional substance having an affinity for a virus that contains a gene useful for gene therapy and an effective amount of another functional substance having an affinity specific for a target cell for which transfer of the gene is required.

The therapeutic composition of the present invention can be prepared by using an effective amount of the above-mentioned functional substance as its active ingredient, and formulating it with a known pharmaceutical carrier. The composition can be administrated as an injectable preparation or a drip.

A dosage of the therapeutic composition is appropriately determined and varies depending on the particular dosage form, administration route and purpose as well as age, weight and conditions of a patient to be treated. In general, a daily dosage for an adult person is 10 µg to 200 mg/kg in terms of the amount of the active ingredient contained in the formulation. Of course, the dosage can vary depending on various factors. Therefore, in some cases, a less dosage than the above may be sufficient but, in other cases, a dosage more than the above may be required.

The present invention provides a gene therapy method comprising administering as an active ingredient an effective amount of the therapeutic composition of the present invention.

In the gene therapy method of the present invention, the cell as a vehicle having an affinity for a retrovirus may be administered being bound to an effective amount of a retrovirus containing a gene useful for gene therapy. Alternatively, a cell as a vehicle having an affinity for a retrovirus may be administered such that it binds to the retrovirus due to its affinity in vivo. In either case, the mode of administration is determined such that a gene is efficiently transferred into target cells in vivo.

Although it is not intended to limit the present invention, it is preferable to select a method for administration suitable for the composition for gene therapy of the present invention to reach target cells.

Examples of cells to be used as a target for gene transfer according to the present invention include, but are not limited to, stem cells, hematopoietic cells, non-adhesive low-density mononuclear cells, adhesive cells, bone marrow cells, hematopoietic stem cells, peripheral blood stem cells, umbilical cord blood cells, fetal hematopoietic stem cells, non-human embryogenic stem cells, non-human embryonic cells, primordial germ cells, oocytes, oogonia, ova, spermatocytes, sperms, CD34+ cells, c-kit+ cells, pluripotent hematopoietic progenitor cells, unipotent hematopoietic progenitor cells, erythroid precursor cells, lymphoid mother cells, mature blood cells, blood cells, leukocytes, lymphocytes, B cells, T cells, tumor-infiltrating lymphocytes, fibroblasts, neuroblasts, neurocytes, endothelial cells, vascular endothelial cells, hepatocytes, myoblasts, skeletal muscle cells, smooth muscle cells, cancer cells, myeloma cells and leukemia cells.

Some of gene therapies using hematopoietic stem cells as target cells are for complementing a deficient or abnormal gene in a patient. Examples thereof include the gene therapy for adenosine deaminase (ADA) deficiency (see United States Patent No. 5399346) and Gaucher's disease. In addition, a drug resistance gene may be transferred into hematopoietic stem cells in order to alleviate the damage of hematopoietic cells due to the chemotherapeutic agents used for the treatment of cancer or leukemia, for example.

Furthermore, a therapeutic method in which a suicide gene such as thymidine kinase gene is transferred into cancer cells and then a drug is administered to kill the cells has been studied as a gene therapy for cancer (Science, 256:1550-1552 (1992)). In addition, attempts are made to treat AIDS using a gene therapy. In this case, the following procedure is considered. In the procedure, a gene encoding a nucleic acid molecule (e.g., an antisense nucleic acid or a ribozyme) which interferes with the replication or the gene expression of human immunodeficiency virus (HIV) is transferred into T cells which can be infected with the causal agent of AIDS, HIV [e.g., J. Virol., 69:4045-4052 (1995)]. The target cell-specific gene transfer according to the present invention can increase the efficiencies of gene therapies as described above.

As described above in detail, a disease that requires gene therapy for its treatment can be treated by specifically transferring a gene into target cells in vivo using the therapeutic composition and the therapeutic method of the present invention. No acute toxicity is observed when the therapeutic composition of the present invention is administered at a physiologically effective concentration into a living body

### Examples

The following Examples illustrate the present invention in more detail, but are not to be construed to limit the scope thereof.

### Example 1

(1) A vector PGK-hADA (Nature Medicine, 2:876-882 (1996)), a PGK vector containing human ADA gene (hADA) produced by EPHA-5-producer cells (Nature Medicine, 2:876-882 (1996)), and a injectable preparation of RetroNectin as described below in Example 2 were injected into caudal vein of a C3H/HeJ mouse (8 weeks old, purchased from Japan SLC). Transduction of hematopoietic stem cells was analyzed as described in Nature Medicine, 2:876-882 (1996) by examining the expression of the transduced human ADA cDNA in the mouse having a transferred gene. Specifically, the presence of human ADA protein in peripheral blood cells from the mouse was confirmed by ADA isozyme analysis which uses cellulose acetate electrophoresis for detection. The examination was conducted at the beginning of the fourth month after the transplantation and repeated every month.
(2) Analysis of transduced bone marrow from the transplanted mouse using the isozyme analysis after nine months confirmed the expression of human ADA cDNA for a mouse administered with RetroNectin and the vector PGK-hADA. Human ADA was not detected for a control mouse.

### Example 2

RetroNectin (CH-296, Takara Shuzo) was dissolved in injectable water at a concentration of 2 mg/ml. The solution was equilibrated with saline to prepare injectable preparations.

### Example 3: Gene transfer using HL-60 cell as vehicle

A polypeptide CH-271 was prepared as follows. Briefly, Escherichia coli HB101/pCH101 (FERM BP-2799) was cultured according to the method as described in United States Patent No. 5,198,423. CH-271 was obtained from the culture.

Human leukemia HL-60 cells (purchased from Dainippon Pharmaceutical) were suspended in D-MEM medium (Bio Whittaker) containing 10% fetal calf serum (FCS, Bio Whittaker) at a concentration 2 x 10⁶ cells/ml. RetroNectin^{™} (Takara Shuzo) or CH-271 was added to 1 ml of the cell suspension at a final concentration of 100 µg/ml. A control group to which no such functional substance was added was provided.

100 µl of a solution containing 6.23 x 10⁶ cfu/ml of an ecotropic retrovirus vector having an enhanced green fluorescent protein (EGFP) gene (pLEIN (Clontech), prepared using GP+E-86 cells (ATCC CRL-9642)) was added to the cell. The mixture was incubated at 37°C for 30 minutes in a 5% CO₂ incubator. After incubation, the cells were washed twice by centrifugation in D-MEM medium containing 10% FCS to remove the virus vector which did not adsorb to the cells. After the washing by centrifugation, the cells were suspended in 1 ml of D-MEM medium containing 10% FCS.

2 x 10⁶ of the thus-obtained HL-60 cells were added to a 6-well cell culture plate (Falcon) in which 2 x 10⁵ of NIH/3T3 cells (ATCC CRL-1658) had been cultured. The cells were incubated at 37°C for 2 days in a 5% CO₂ incubator. After incubation, NIH/3T3 cells adhered to the plate were collected. EGFP-expressing cells were analyzed by flow cytometry using FACSVantage (Becton Dickinson) at an excitation wavelength of 488 nm and an emission wavelength of 515-545 nm. The gene transfer efficiency (the ratio of EGFP-expressing cells to total cells) was then calculated. The experimental results are shown in Figure 1.

As shown in Figure 1, significant expression of the EGFP gene derived from the GP+E-86/EGFP retrovirus vector was observed only for the group in which RetroNectin (CH-296) was added to HL-60 cells, indicating that gene transfer took place. Specifically, it was demonstrated that the retrovirus vector could be adsorbed to HL-60 cells via RetroNectin, approach NIH/3T3 cells as target cells along with HL-60 cells, and then infect the target cells. The virus vector adsorbed to the cells via RetroNectin was not detached after centrifugation or washing, and did not lose its infectivity upon adsorption. As described above, a vehicle cell capable of adsorbing a virus could be conveniently prepared only by adding RetroNectin to a cell suspension.

RetroNectin has a ligand (CS-1) for VLA-4, which is expressed on HL-60 cells, in addition to a ligand for VLA-5. On the other hand, CH-271 has a ligand for VLA-5 of which the expression level on HL-60 is low. It is considered that this difference reflects the difference in the gene transfer efficiency. These results show that it is possible to specifically confer an affinity for a virus on the vehicle cells of interest even in a state in which plural types of cells coexist by appropriately selecting a functional substance having an affinity for the virus to be used in combination with the cells (e.g., a fibronectin fragment).

### Example 4: Gene transfer using vascular endothelial cell as vehicle

RetroNectin was added at a final concentration of 100 µg/ml to 200 µl of D-MEM medium (Bio Whittaker, supplemented with 10% FBS) containing 5 x 10⁵ of vascular endothelial cells (HUVECs, purchased from Bio Whittaker). A control group to which RetroNectin was not added was provided.

200 µl of a solution containing GP+E-86/EGFP retrovirus at a concentration of 7.75 x 10⁶ cfu/ml was added to the cells. The mixture was incubated at 37°C for 30 minutes in a 5% CO₂ incubator. After incubation, the cells were washed twice by centrifugation in D-MEM medium containing 10% FCS to remove the virus vector which did not adsorb to the cells. After the washing by centrifugation, the cells were suspended in 100 µl of D-MEM medium containing 10% FCS. 5 x 10⁵ of HUVEC cells prepared as described above were mixed with 1 x 10⁵ L1210 cells (purchased from Dainippon Pharmaceutical) in 500 µl of RPMI 1640 medium (Bio Whittaker, supplemented with 10% FBS) and transferred to a 24-well plate (Falcon). The cells were incubated at 37°C for 4 days in a 5% CO₂ incubator.

When the cells were examined under a fluorescence microscope after cultivation, a cluster of HUVECs surrounded by L1210 cells was observed, demonstrating that HUVECs had an affinity for L1210 cells. Furthermore, fluorescence from EGFP was observed for L1210 cells surrounding HUVECs, demonstrating that gene transfer took place in these cells. For the cells subjected to the above-mentioned procedure without the addition of RetroNectin, surrounding of HUVECs by L1210 cells was observed, but fluorescence was not observed.

From the above, it was demonstrated that a gene can be targeted into target cells using HUVECs and a functional substance such as RetroNectin in combination.

### Example 5: Homing of vascular endothelial cell

Using Adenovirus Expression Vector Kit (Takara Shuzo), an adenovirus vector AxCAiLacZ which contains a control plasmid having a lacZ gene attached to the kit, pAxCAiLacZ, was prepared. HUVECs cultured in a 10-cm plate (Falcon) almost to confluence were infected with the adenovirus vector AxCAiLacZ at m.o.i. = 10, and the cultivation was continued. The cells collected 3 days after the infection were designated as LacZ-HUVECs.

1 x 10⁶ cells of a mouse fibrosarcoma cell line Meth-A (distributed by the Institute of Physical and Chemical Research (RIKEN), RCB 0464) were subcutaneously transplanted into a SCID mouse (obtained from Clea Japan). 2 x 10⁶ of LacZ-HUVECs were inoculated through caudal vein 5 days after the transplantation. Tumor, peritoneum (together with abdominal wall) for which vascularization was observed at sites adjacent to the tumor, and various organs (liver, spleen, heart, kidney and lung) were removed from the mouse 7 days after the inoculation. Each of the above was stained using X-gal (Takara Shuzo) to examine the localization of LacZ-HUVECs. Furthermore, a portion of LacZ-HUVECs was subjected to cultivation in a plate at the same time of the inoculation into the mouse and stained with X-gal when the tumor, peritoneum and organs were stained in order to confirm that LacZ-HUVECs had the lacZ gene.

LacZ-HUVECs cultured in the plate were stained blue with X-gal, confirming that they harbored the lacZ gene. Portions of the removed tumor and peritoneum were stained blue with X-gal. In particular, a line-shaped blue staining was observed along the sites of vascularization in the peritoneum, confirming that LacZ-HUVECs were localized at the sites of vascularization. In addition, blue staining was also observed for a portion of the tumor. Thus, it was demonstrated that the administered HUVECs were selectively accumulated at the sites of vascularization.

As described above, it was demonstrated that HUVECs can be used as a vehicle for transferring a gene to a site of tumorigenesis and a site of vascularization.

### Industrial Applicability

The present invention provides a therapy which enables targeting of gene transfer into target cells in vivo, which transfers a gene specifically into target cells of interest, and, consequently, which is useful for treatment of diseases susceptible to gene therapy, as well as a composition for the therapy. Furthermore, the present invention provides a gene therapy method comprising administering said therapeutic composition and a gene therapy method comprising transferring a gene into target cells in vivo.

## Claims

1. A composition for gene therapy used for treating a disease susceptible to gene therapy, which contains as active ingredients an effective amount of a retrovirus that contains a gene useful for gene therapy as well as an effective amount of a cell as a vehicle that has an affinity for the retrovirus and an affinity specific for a target cell for which transfer of the gene is required, wherein the affinity for the retrovirus is derived from a functional substance selected from the group consisting of anti-virus antibodies, heparin-II-binding domain of fibronectin, fibroblast growth factor, collages and polylysine, and is conferred by binding said functional substance to the cell as a vehicle or by expressing said functional substance on the surface of the cell as a vehicle.

2. The composition according to claim 1, wherein the affinity for the target cell is conferred by forcing the cell as a vehicle to express a functional substance having the affinity for the target cell on said cell as a vehicle, wherein said functional substance is a ligand for a receptor expressed on the target cell or an antibody against a surface antigen on the target cell.

3. The composition according to claim 1 or 2, wherein the cell as a vehicle is selected from the group consisting of vascular endothelial cells, inflammatory cells, hematopoietic stem cells, brain endothelial cells and bone marrow cells.

4. Use of an effective amount of a cell as a vehicle that has an affinity for a retrovirus that contains a gene useful for gene therapy and an affinity specific for a target cell for which transfer of the gene is required for the preparation of a pharmaceutical composition for gene therapy for treating a disease susceptible to gene therapy, wherein the affinity for the retrovirus is derived from a functional substance selected from the group consisting of anti-virus antibodies, heparin-II-binding domain of fibronectin, fibroblast growth factor, collagen and polylysine, and is conferred by binding said functional substance to the cell as a vehicle or by expressing said functional substance on the surface of the cell as a vehicle.

5. The use according to claim 4, wherein the affinity for the target cell is conferred by forcing the cell as a vehicle to express a functional substance having the affinity for the target cell on said cell has a vehicle, wherein said functional substance is a ligand for a receptor expressed on the target cell or an antibody against a surface antigen on the target cell.

6. The use according to claim 4 or 5, wherein the composition contains an effective amount of the retrovirus that contains a gene useful for gene therapy.

7. The use according to any one of claims 4 to 6, wherein the cell as a vehicle is selected from the group consisting of vascular endothelial cells, inflammatory cells, hematopoietic stem cells, brain endothelial cells and bone marrow cells.

8. The composition or the use according to any one of claims 1 to 7, wherein the target cell is a cell selected from the group consisting of hematopoietic stem cells, blood cells, leukocytes, lymphocytes, T cells, tumor-infiltrating lymphocytes, B cells and cancer cells.

9. The composition or the use according to any one of claims 1 to 3 and 6 to 8, wherein the gene useful for gene therapy encodes a therapeutic protein which is expressed upon expression of the gene in the target cell in an amount sufficient for the treatment.

10. The composition or the use according to claim 9, wherein the therapeutic protein is an enzyme or a cytokine.

11. The composition or the use according to any one of claims 1 to 3 and 6 to 10, wherein the retrovirus is a retrovirus vector.

## Patentansprüche

1. Zusammensetzung zur Gentherapie, die zur Behandlung einer der Gentherapie zugänglichen Krankheit verwendet wird und die als Wirkstoffe eine wirksame Menge eines Retrovirus, das ein für Gentherapie verwendbares Gen enthält, sowie eine wirksame Menge einer Zelle als Träger enthält, die eine Affinität für das Retrovirus und eine Affinität, die für eine Zielzelle, für die ein Transfer des Gens benötigt wird, spezifisch ist, aufweist, wobei die Affinität für das Retrovirus von einer funktionellen Substanz abgeleitet ist, ausgewählt aus der Gruppe, bestehend aus Anti-Virus-Antikörpern, Heparin II-bindender Domäne von Fibronectin, Fibroblasten-Wachstumsfaktor, Kollagen und Polylysin, und durch Binden der funktionellen Substanz an die Zelle als Träger oder durch Expression der funktionellen Substanz an der Oberfläche der Zelle als Träger verliehen wird.

2. Zusammensetzung gemäß Anspruch 1, wobei die Affinität für die Zielzelle **dadurch** verliehen wird, dass die Zelle als Träger dazu veranlasst wird, eine funktionelle Substanz, die die Affinität für die Zielzelle aufweist, auf der Zelle als Träger zu exprimieren, wobei die funktionelle Substanz ein Ligand für einen auf der Zielzelle exprimierten Rezeptor oder ein Antikörper gegen ein Oberflächen-Antigen auf der Zielzelle ist.

3. Zusammensetzung gemäß Anspruch 1 oder 2, wobei die Zelle als Träger ausgewählt ist aus der Gruppe, bestehend aus vaskulären Endothelzellen, Entzündungszellen, hämatopoetischen Stammzellen, Endothelzellen des Gehirns und Knochenmarkzellen.

4. Verwendung einer wirksamen Menge einer Zelle als Träger, die eine Affinität für ein Retrovirus, das ein für Gentherapie verwendbares Gen enthält, und eine Affinität, die für eine Zielzelle, für die ein Transfer des Gens benötigt wird, spezifisch ist, aufweist, für die Herstellung einer pharmazeutischen Zusammensetzung für Gentherapie zur Behandlung einer der Gentherapie zugängliche Krankheit, wobei die Affinität für das Retrovirus von einer funktionellen Substanz abgeleitet ist, ausgewählt aus der Gruppe, bestehend aus Anti-Virus-Antikörpern, Heparin II-bindender Domäne von Fibronectin, Fibroblasten-Wachstumsfaktor, Kollagen und Polylysin, und durch Binden der funktionellen Substanz an die Zelle als Träger oder durch Expression der funktionellen Substanz an der Oberfläche der Zelle als Träger verliehen wird.

5. Verwendung gemäß Anspruch 4, wobei die Affinität für die Zielzelle **dadurch** verliehen wird, dass die Zelle als Träger dazu veranlasst wird, eine funktionelle Substanz, die die Affinität für die Zielzelle aufweist, auf der Zelle als Träger zu exprimieren, wobei die funktionelle Substanz ein Ligand für einen auf der Zielzelle exprimierten Rezeptor oder ein Antikörper gegen ein Oberflächen-Antigen auf der Zielzelle ist.

6. Verwendung gemäß Anspruch 4 oder 5, wobei die Zusammensetzung eine wirksame Menge des Retrovirus enthält, das ein für Gentherapie verwendbares Gen enthält.

7. Verwendung gemäß einem der Ansprüche 4 bis 6, wobei die Zelle als Träger ausgewählt ist aus der Gruppe, bestehend aus vaskulären Endothelzellen, Entzündungszellen, hämatopoetischen Stammzellen, Endothelzellen des Gehirns und Knochenmarkszellen.

8. Zusammensetzung oder Verwendung gemäß einem der Ansprüche 1 bis 7, wobei die Zielzelle eine Zelle ist, ausgewählt aus der Gruppe, bestehend aus hämatopoetischen Stammzellen, Blutzellen, Leukozyten, Lymphozyten, T-Zellen, Tumor-infiltrierenden Lymphozyten, B-Zellen und Krebszellen.

9. Zusammensetzung oder Verwendung gemäß einem der Ansprüche 1 bis 3 und 6 bis 8, wobei das für Gentherapie verwendbare Gen ein therapeutisches Protein kodiert, das bei Expression des Gens in der Zielzelle in einer Menge exprimiert wird, die für die Behandlung ausreichend ist.

10. Zusammensetzung oder Verwendung gemäß Anspruch 9, wobei das therapeutische Protein ein Enzym oder ein Cytokin ist.

11. Zusammensetzung oder Verwendung gemäß einem der Ansprüche 1 bis 3 und 6 bis 10, wobei das Retrovirus ein Retrovirus-Vektor ist.

## Revendications

1. Composition pour thérapie génique utilisée pour traiter une maladie se prêtant à une thérapie génique, qui contient comme principes actifs une quantité efficace d'un rétrovirus qui contient un gène utilisable pour une thérapie génique ainsi qu'une quantité efficace d'une cellule en tant que véhicule qui a une affinité pour le rétrovirus et une affinité spécifique pour une cellule cible pour laquelle le transfert de gène est nécessaire, dans laquelle l'affinité pour le rétrovirus provient d'une substance fonctionnelle choisie dans le groupe constitué d'anticorps anti-virus, domaine se liant à l'héparine II de la fibronectine, facteur de croissance fibroblastique, collagène et polylysine, et est conférée par la liaison de ladite substance fonctionnelle à la cellule véhicule ou par l'expression de ladite substance fonctionnelle sur la surface de la cellule véhicule.

2. Composition selon la revendication 1, dans laquelle l'affinité pour la cellule cible est conférée en forçant la cellule véhicule à exprimer une substance fonctionnelle ayant l'affinité pour la cellule cible sur ladite cellule véhicule, dans laquelle ladite substance fonctionnelle est un ligand pour un récepteur exprimé sur la cellule cible ou un anticorps contre un antigène de surface sur la cellule cible.

3. Composition selon la revendication 1 ou 2, dans laquelle la cellule véhicule est choisie dans le groupe constitué de cellules endothéliales vasculaires, cellules inflammatoires, cellules souches hématopoïétiques, cellules endothéliales cérébrales et cellules de moelle osseuse.

4. Utilisation d'une quantité efficace d'une cellule véhicule qui a une affinité pour un rétrovirus qui contient un gène utilisable pour une thérapie génique et une affinité spécifique pour une cellule cible pour laquelle le transfert du gène est nécessaire pour la préparation d'une composition pharmaceutique pour une thérapie génique pour le traitement d'une maladie se prêtant à une thérapie génique, dans laquelle l'affinité pour le rétrovirus provient d'une substance fonctionnelle choisie dans le groupe constitué d'anticorps anti-virus, domaine se liant à l'héparine II de la fibronectine, facteur de croissance fibroblastique, collagène et polylysine, et est conférée par la liaison de ladite substance fonctionnelle à la cellule véhicule ou par l'expression de ladite substance fonctionnelle sur la surface de la cellule véhicule.

5. Utilisation selon la revendication 4, dans laquelle l'affinité pour la cellule cible est conférée en forçant la cellule véhicule à exprimer une substance fonctionnelle ayant l'affinité pour la cellule cible sur ladite cellule véhicule, dans laquelle ladite substance fonctionnelle est un ligand pour un récepteur exprimé sur la cellule cible ou un anticorps contre un antigène de surface sur la cellule cible.

6. Utilisation selon la revendication 4 ou 5, dans laquelle la composition contient une quantité efficace du rétrovirus qui contient un gène utilisable pour une thérapie génique.

7. Utilisation selon l'une quelconque des revendications 4 à 6, dans laquelle la cellule véhicule est choisie dans le groupe constitué de cellules endothéliales vasculaires, cellules inflammatoires, cellules souches hématopoïétiques, cellules endothéliales cérébrales et cellules de moelle osseuse.

8. Composition ou utilisation selon l'une quelconque des revendications 1 à 7, dans laquelle la cellule cible est une cellule choisie dans le groupe constitué de cellules souches hématopoïétiques, cellules sanguines, leucocytes, lymphocytes, lymphocytes T, lymphocytes infiltrant les tumeurs, lymphocytes B et cellules cancéreuses.

9. Composition ou utilisation selon l'une quelconque des revendications 1 à 3 et 6 à 8, dans laquelle le gène utilisable pour une thérapie génique code pour une protéine thérapeutique qui est exprimée lors de l'expression du gène dans la cellule cible en une quantité suffisante pour le traitement.

10. Composition ou utilisation selon la revendication 9, dans laquelle la protéine thérapeutique est une enzyme ou une cytokine.

11. Composition ou utilisation selon l'une quelconque des revendications 1 à 3 et 6 à 10, dans laquelle le rétrovirus est un vecteur rétroviral.
